# EUROPEAN PATENT APPLICATION

(11) **EP 1 514 547 A1**
(43) Date of publication of application: **16.03.2005**
(21) Application number: 03733434.9
(22) Date of filing: 16.06.2003
(51) Int. Cl.: A61K 31/47, A61P 3/06, A61P 43/00

(54) **CONTROLLED-RELEASE DRUG COMPOSITION**

(30) Priority: 17.06.2002 US 388740 P
(71) Applicant: Kowa Co., Ltd., Naka-ku, Nagoya-shi, Aichi 460-8625 (JP); Nissan Chemical Industries, Ltd., Chiyoda-ku, Tokyo 101-0054 (JP)
(72) Inventor: TANIZAWA, Yoshio, FUJI-SHI, Shizuoka 416-0947 (JP); SHIMOKAWA, Tatsuharu, Shizuoka 416-0908 (JP); OGAWA, Hirotada, FUJI-SHI, Shizuoka 417-0014 (JP); WATANABE, Mayumi, Shizuoka 417-0001 (JP); KOZAKI, Chihiro, Shizuoka 417-0841 (JP); KAWASHIMA, Hiroyuki, FUJI-SHI, Shizuoka 417-0014 (JP); SHINODA, Yasuo, SHIZUOKA-SHI, Shizuoka 420-0867 (JP); INAGI, Toshio, MISHIMA-SHI, Shizuoka 411-0038 (JP)
(74) Representative: Hartz, Nikolai, Dr.
(86) International application number: PCT/JP2003/007605
(87) International publication number: WO 2003/105848

(57) **Abstract**

The present invention is drawn to a pharmaceutical composition characterized by containing a composition (A) which contains pitavastatin, a salt thereof, or an ester thereof and which initiates release thereof at least in the stomach, and an enteric composition (B) which contains pitavastatin, a salt thereof, or an ester thereof. By use of the controlled release pharmaceutical compositlon of the present invention, the blood level of pitavastatin can be maintained at an appropriate level immediately after administration over a long period of time. Thus, highly safety and effective treatment of hypercholesterolemia can be performed.

## Description

### Technical Field

The present invention relates to a controlled release pharmaceutical composition containing pitavastatin, which is an HMG-CoA reductase inhibitor, a salt thereof, or an ester thereof.

### Background Art

Pitavastatin and salts and esters thereof are known to be useful as drugs for treating hypercholesterolemia, by virtue of their excellent HMG-CoA reductase inhibitory activity (USP 5,856,336 and EP 0,304,063). Hypercholesterolemia treatment drugs such as the pitavastatin-containing drugs are used in the form of peroral preparations such as tablets, granules and capsules (USP 6,465,477 and WO 97/23200). In general, peroral drug preparations are designed such that the blood level of the active ingredient reaches its peak 0.5 to 3 hours after ingestion, and the level rapidly decreases afterward. However, since cholesterol is synthesized in the body during the period from midnight to morning, it is highly likely that blood level of the active ingredient does not match the time at which biosynthesis of cholesterol occurs.

Although pitavastatin and salts and esters thereof are also known to exert high activity and have high safety, an excessively high blood level thereof is not preferred, from the viewpoint of prevention against the side effects.

In addition, it is also desired for pitavastatin and salts and esters thereof to maintain an excellent hypercholesterol-reducing action for a long period of time.

In view of the foregoing, an object of the present invention is to provide a controlled release pharmaceutical composition which can reliably maintain an appropriate blood level of pitavastatin, a salt thereof, or an ester thereof over a long period of time.

### Disclosure of the Invention

The present inventors have carried out extensive studies in order to develop a controlled release pharmaceutical composition containing pitavastatin, a salt thereof, or an ester thereof. A first approach to resolution of the aforementioned problems is a sustained release drug preparation exhibiting zero-order drug release. However, when such a sustained release drug preparation exhibiting zero-order drug release is administered singly, increasing the blood level thereof immediately after administration is difficult, resulting in a considerably low level of the maximum blood concentration (Cₘₐₓ) as compared, with ordinary drug preparations of the same dose. In addition, the area under the blood concentration curve (AUC)―an index of hypercholesterol-reducing action―decreases considerably. Thus, the first approach fails to resolve the aforementioned problems.

Consequently, the present inventors have studied the absorption kinetics of pitavastatin and salts and esters thereof, and have found that pitavastatin and salts and esters thereof are absorbed most effectively in the duodenum and also effectively in the large intestine as well as the small intestine, which contrasts greatly with the general tendency for ordinary drugs to be absorbed in the small intestine. On the basis of this finding, the present inventors have conducted further studies, and have found that when a drug preparation is formed from a composition which contains pitavastatin, a salt thereof, or an ester thereof and which initiates release thereof at least in the stomach and, in combination, an enteric composition which contains pitavastatin, a salt thereof, or an ester thereof, the drug preparation can attain an appropriate effective blood level thereof immediately after administration and can maintain such an appropriate blood level for a long period of time. The present invention has been accomplished on the basis of these findings.

Accordingly, the present invention provides a pharmaceutical composition characterized by comprising a composition (A) which contains pitavastatin, a salt thereof, or an ester thereof and which initiates release thereof at least in the stomach, and an enteric composition (B) which contains pitavastatin, a salt thereof, or an ester thereof.

### Brief Description of the Drawings

Fig. 1 shows the dissolution test results of the compositions of Example 5 and Comparative Examples 1 and 2.
Fig. 2 shows the change in blood plasma level of pitavastatin in the case where pitavastatin was perorally administered in dogs, in the form of the compositions of Example 5 and Comparative Examples 1 and 2.

### Best Mode for Carrying Out the Invention

The characteristic feature of the pharmaceutical composition of the present invention is that the composition comprises a composition (A) which contains pitavastatin, a salt thereof, or an ester thereof (hereinafter these compounds are collectively referred to as pitavastatin) and which initiates release of pitavastatin at least in the stomach, and an enteric composition (B) which contains pitavastatin. Herein, the composition (A) initiates release of pitavastatin at least in the stomach. The composition (A) may release, in the stomach, the practical portion of pitavastatin or a portion of pitavastatin. However, a composition (A) which releases at least 30 mass% pitavastatin in the stomach is preferred, from the viewpoint of reliable attainment of an effective blood level of pitavastatin immediately after administration.

Preferably, the enteric composition of (B) is a composition prepared by coating pitavastatin or a pitavastatin-containing composition with a component which dissolves at a pH of 3.0 or higher. Alternatively, a composition prepared by mixing pitavastatin with a component which dissolves at a pH of 3.0 or higher is preferred for the enteric composition of (B). Accordingly, the portion of pitavastatin contained in the enteric composition (B) is hardly released in the stomach but is released after passing through the near area of the duodenum.

One characteristic of pitavastatin, which serves an active ingredient of the controlled release pharmaceutical composition in the present invention, is high absorption thereof in the duodenum (see Table 1). Thus, the practical portion of pitavastatin released from the composition (A) in the stomach is considered to be absorbed in the duodenum. In contrast, the enteric composition (B) releases pitavastatin after passing through the near area of the duodenum. Thus, pitavastatin released from the composition (B) is firstly absorbed in the small intestine. The percentage of pitavastatin absorption in the small intestine is about one-third that in the duodenum (see Table 1). Therefore, even if pitavastatin is rapidly released in the small intestine, the abrupt absorption of pitavastatin does not occur. Furthermore, pitavastatin requires a relatively long period of time for the passage thereof through these organs. Thus, pitavastatin is slowly absorbed in sites; i.e., from the small intestine to the large intestine, over a relatively long time. Therefore, the controlled release pharmaceutical composition of the present invention can maintain a desirable blood level of pitavastatin.

Since the absorption site in digestive tract of ordinary drugs is the small intestine, a drug which has passed through the small intestine and has reached the large intestine is hardly absorbed. In this connection, sustained release drug preparations involve a problem, for example, in that bioavailability of the drug is reduced as compared with ordinary drug preparations of the same dose. In contrast, pitavastatin exhibits high absorption in the large intestine as well as in the duodenum (see Table 1). Therefore, the controlled release pharmaceutical composition of the present invention can attain a virtually identical level of bioavailability as compared with ordinary drug preparations of the same dose.

**Table 1**

| Absorption sites of pitavastatin in rat (in situ loop test) | | |
|---|---|---|
| Sites | Absorption percentage (%) | |
| | 0.5 h | 1 h |
| Stomach | 8.6 ± 1.1 | 14.4 ± 1.6 |
| Duodenum | 65.9 ± 2.1 | 60.3 ± 10.2 |
| Small intestine | 23.1 ± 3.8 | 21.3 ± 3.0 |
| Large intestine | 34.4 ± 3.7 | 51.6 ± 6.7 |

Pitavastatin which is used in the controlled release pharmaceutical composition of the present invention is a compound denominated (+)-(3R, 5S, 6E)-7-[2-cyclopropyl-4-(4-fluorophenyl)-3-quinolyl]-3,5-dihydroxy-6-heptenoic acid. As mentioned above, the compound is known to be an excellent HMG-CoA reductase inhibitor and to be useful as a drug for treating hypercholesterolemia. Examples of preferred pitavastatin salts includes salts of alkali metal (e.g., sodium or potassium) and salts of alkaline earth metal (e.g., calcium or magnesium). Of these, calcium salt is particularly preferred. Examples of preferred pitavastatin esters include alkyl esters such as a methyl ester, an ethyl ester, an i-propyl ester, and an n-propyl ester.

As mentioned above, the composition (A) preferably releases in the stomach at least 30 mass% pitavastatin contained in the composition (A). More preferably, the amount of pitavastatin released in the stomach is controlled such that the composition (A) releases, in the stomach, for example, at least 30 mass% and less than 60 mass% pitavastatin, at least 60 mass% and less than 85 mass% pitavastatin, at least 85 mass% pitavastatin contained in the composition (A). Herein, the percentage of pitavastatin release from the composition (A) in the stomach is determined in accordance with General Test Methods in Japanese Pharmacopoeia, Dissolution Test Method No.2 (Paddle method). Specifically, the composition (A) to be tested is added to artificial gastric juice medium (pH: 1.2; 900 mL), and the medium is stirred for 30 minutes at 37 ± 1°C with a paddle rotation of 100 rpm. After termination of stirring, the amount of pitavastatin dissolved in artificial gastric juice medium is determined. Alternatively, in accordance with General Test Methods in Japanese Pharmacopoeia, Dissolution Test Method No. 2 (Paddle method), a pharmaceutical composition to be tested is added to artificial gastric juice medium (pH: 1.2; 900 mL), and the medium is stirred for 30 minutes at 37 ± 1°C with a paddle rotation of 100 rpm. After termination of stirring, the amount of pitavastatin dissolved in artificial gastric juice medium is determined. The percentage of pitavastatin release from the composition (A) is determined through calculation by use of the amount of pitavastatin contained the composition (A) included in the pharmaceutical composition to be tested.

The percentage of pitavastatin release from the composition (A) in the stomach can be regulated by adding a sustained release component to an ordinary employed base. Examples of the bases which can be incorporated into the composition (A) include diluents such as lactose, corn starch, modified corn starch, mannitol, sorbitol, wood cellulose, microcrystalline cellulose, and calcium carbonate; binders such as hydroxypropyl cellulose, hydroxypropylmethyl cellulose, polyvinylpyrrolidone, and partially hydrolyzed polyvinyl alcohol; disintegrants such as low-substituted hydroxypropyl cellulose, carmellose, sodium carboxystarch, carmellose calcium, corn starch, partial pregelatinized starch, croscarmellose sodium, and crospovidone; lubricants such as magnesium stearate, stearic acid, palmitic acid, calcium stearate, talc; coating agents such as saccharides, cellulose derivatives, polyvinyl derivatives, alkylene oxide polymers, greases, methyl methacrylate·butyl methacrylate·dimethylaminoethyl methacrylate copolymers, acacia, locust bean gum, carrageenan, xanthan gum, pregelatinized starch, pectin, glucomannan, gluten, casein, gelatin, and zein.

Examples of the saccharides include lactose, fructose, glucose, sucrose, maltose, sorbitol, xylitol, maltitol, mannitol, trehalose, cyclodextrin, erythritol, hydrogenated palatinose, and lactitol.

Examples of the cellulose derivatives include methylcellulose, ethylcellulose, hydroxypropylcellulose, hydroxypropylmethylcellulose, and low-substituted hydroxypropylcellulose.

Examples of the polyvinyl derivatives include polyvinylpyrrolidone, polyvinylacetal diethylaminoacetate, and partially hydrolyzed polyvinyl alcohol.

Examples of the alkylene oxide polymers include polyethylene glycol and polypropylene glycol.

Examples of the greases include sucrose esters of fatty acids and polyoxyl 40 stearate.

In order to enhance the time-lapsing stability of pitavastatin, a basic substance which can elevate pH of an aqueous solution or dispersion of the composition (A) to 6.8 or higher, particularly 6.8 to 7.8 is preferably added to the composition (A). Examples of such basic substances include antacids such as magnesium aluminometasilicate, magnesium aluminosilicate, magnesium aluminate, dried aluminum hydroxide, synthetic hydrotalcite, synthetic aluminum silicate, magnesium carbonate, precipitated calcium carbonate, magnesium oxide, aluminum hydroxide, and sodium hydrogencarbonate; and pH-adjuster agent such as L-arginine, sodium phosphate, disodium hydrogenphosphate, sodium dihydrogenphosphate, potassium phosphate, dipotassium hydrogenphosphate, potassium dihydrogenphosphate, disodium citrate, sodium succinate, ammonium chloride, and sodium benzoate. Among them, magnesium aluminometasilicate, L-arginine, and dipotassium hydrogenphosphate are particularly preferably used.

As used herein, the "pH" means a pH value of a 5 w/v% suspension containing a unit amount of the pharmaceutical composition of the present invention or each of compositions (composition (A) or enteric composition (B)) which is administered.

Preferably, the sustained release component is incorporated into the composition (A) in such a manner that pitavastatin or a pitavastatin-containing composition is coated with the sustained release component, or that pitavastatin and the sustained release component are mixed. Examples of the sustained release components for coating include biodegradable polymers, cellulose derivatives, (meth)acrylic acid (co)polymers, alkylene oxide polymers, greases, silicones, chitin, chitosan, casein, tragacanth gum, guar gum, gellan gum, and gum acacia.

Examples of the biodegradable polymers include poly(lactic acid), poly(glycolic acid), poly(hydroxybutyric acid), poly-α-cyanoacrylate ester, poly(ortho ester), poly(amino acid), and gelatin.

Examples of the cellulose derivatives include methylcellulose, ethylcellulose, propylcellulose, hydroxymethylcellulose, hydroxyethylcellulose, hydroxypropylcellulose, hydroxypropylmethylcellulose, carboxymethylcellulose, carboxyethylcellulose, carboxypropylcellulose, methylhydroxypropylcellulose, cellulose acetyl phthalate, cellulose diacetyl phthalate, cellulose triacetyl phthalate, polyoxyethylcellulose phthalate, hydroxyethylcellulose phthalate, hydroxypropylcellulose phthalate, cellulose acetate, and salts thereof.

Examples of the (meth)acrylic acid (co)polymers include ethyl acrylate·methyl methacrylate·(chlorotrimethylammonio)ethyl methacrylate·copolymers, butyl methacrylate·dimethylaminoethyl methacrylate·copolymers, acrylic acid, methyl acrylate polymers, and methacrylate·(dimethylaminoethyl) ether polymers.

Examples of the alkylene oxide polymers include polyethylene glycol and polypropylene glycol.

Examples of the greases include hydrogenated oil, monoglycerides, triglycerides, waxes, higher fatty acids, sucrose esters of fatty acids, and higher fatty acid glycerin esters.

Examples of the silicones include dimethylpolysiloxane, methylpolysiloxane, and silicone oil; and dimethylpolysiloxane mixtures such as dimethylpolysiloxane-silicon dioxide mixtures, silicone antiform agents, and silicone resin emulsions.

Examples of the sustained release components to be mixed include biodegradable polymers, starches, dextrans, cellulose derivatives, (meth)acrylic acid (co)polymers, alkylene oxide polymers, greases, carrageenan, chitin, chitosan, casein, tragacanth gum, guar gum, gellan gum; paraffin, silicones, acacia, poly(glutamic acid), poly(aspartic acid), polylysine, polyarginine, alginic acid, pectic acid, and xanthan gum.

Examples of the biodegradable polymers include poly(lactic acid), poly(glycolic acid), poly(hydroxybutyric acid), poly-α-cyanoacrylate ester, poly(ortho ester), poly(amino acid), gelatin, collagen, chondroitin sulfate, hyaluronic acid, albumin, casein, globulin, and gluten.

Examples of the starches include α-amylo-starch, gelatinized starch, carboxymethyl starch, carboxyethyl starch, starch phosphate, acid treated starch, oxidized starch, dialdehyde starch, thin-boiling starch, and dextrin.

Examples of the dextrans include dextran, dextran sulfate, and carboxymethyl dextran.

Examples of the cellulose derivatives include methylcellulose, ethylcellulose, hydroxymethylcellulose, hydroxyethylcellulose, hydroxypropylcellulose, carboxyethylcellulose, carboxypropylcellulose, methylhydroxypropylcellulose, cellulose acetyl phthalate, cellulose diacetyl phthalate, cellulose triacetyl phthalate, polyoxyethylcellulose phthalate, hydroxyethylcellulose phthalate, hydroxypropylcellulose phthalate, cellulose acetate, and salts thereof.

Examples of the (meth)acrylic acid (co)polymers include ethyl acrylate·methyl methacrylate·(chlorotrimethylammonio)ethyl methacrylate copolymers, butyl methacrylate·dimethylaminoethyl methacrylate copolymers, acrylic acid, methyl acrylate polymers, and methacrylate·(dimethylaminoethyl) ether polymers.

Examples of the alkylene oxide polymers include polyethylene glycol and polypropylene glycol.

Examples of the greases include hydrogenated oil, hydrogenated castor oil, olive oil, monoglycerides, triglycerides, waxes, higher fatty acids, sucrose esters of fatty acids, higher alcohols, and higher fatty acid glycerin esters.

Examples of the silicones include dimethylpolysiloxane, methylpolysiloxane, and silicone oil; and dimethylpolysiloxane mixtures such as dimethylpolysiloxane-silicon dioxide mixtures, silicone antiform agents, and silicone resin emulsions.

The enteric composition (B) is preferably obtained by coating pitavastatin or a pitavastatin-containing composition with a component which dissolves at a pH of 3.0 or higher, preferably 4.0 or higher, more preferably 5.0 or higher, or by mixing pitavastatin with a component which dissolves at a pH of 3.0 or higher, preferably 4.0 or higher, more preferably 5.0 or higher. Examples of the components which dissolve at a pH of 3.0 or higher include enteric cellulose derivatives, enteric (meth)acrylic acid (co)polymers, enteric maleic acid copolymers, and enteric polyvinyl derivatives. Notably, these components which dissolve at a pH of 3.0 or higher must have no solubility in artificial gastric juice medium having a pH of 1.2.

Examples of the enteric cellulose derivatives include cellulose acetate phthalate, cellulose acetate succinate, cellulose acetate maleate, cellulose benzoate phthalate, cellulose propionate phthalate, cellulose acetate trimellitate, cellulose acetate tetrahydrophthalate, methyl cellulose phthalate, ethylhydroxyethyl cellulose phthalate, hydroxypropylmethyl cellulose acetate phthalate, hydroxypropylmethyl cellulose phthalate, hydroxymethylethyl cellulose phthalate, and carboxymethylethyl cellulose.

Examples of the enteric (meth)acrylic acid (co)polymers include styrene·acrylate copolymers, methyl acrylate·acrylate copolymers, methyl acrylate·copolymers, butyl acrylate·styrene·acrylate copolymers, methacrylic acid·methyl methacrylate copolymers, methacrylic acid·ethyl acrylate copolymers, methyl acrylate·methacrylic acid·octyl acrylate copolymers.

Examples of the enteric maleic acid copolymers include vinyl acetate·maleic anhydride copolymers, styrene·maleic anhydride copolymers, styrene·maleic acid monoester copolymers, vinyl methyl ether·maleic anhydride copolymers, ethylene·maleic anhydride copolymers, vinyl butyl ether·maleic anhydride copolymers, acrylonitrile·methyl acrylate·maleic anhydride copolymers, and butyl acrylate·styrene·maleic anhydride copolymers.

Examples of the enteric polyvinyl derivatives include polyvinyl alcohol phthalate, polyvinyl acetate phthalate, polyvinyl butyrate phthalate, and polyvinyl acetacetate phthalate.

To the enteric composition (B), there may be further added any of the sustained release components, diluents, binders, disintegrants, lubricants, and basic substances, which are described in relation to the components which can be incorporated into the aforementioned composition (A).

The controlled release pharmaceutical composition of the present invention comprises the composition (A) and the enteric composition (B). The ratio by mass of pitavastatin contained in the composition (A) to pitavastatin contained in the enteric composition (B) is adjusted such that the Cₘₐₓ of pitavastatin upon peroral administration of the controlled release pharmaceutical composition of the present invention, and can be appropriately controlled; such that reduction in AUC can be prevented; and such that a constant blood level of pitavastatin can be attained over a long period of time. As used herein, the term "appropriately controlled Cₘₐₓ" refers to a maximum blood level of pitavastatin which is obtained when a composition (A) containing 1 to 8 mg of pitavastatin without sustained release component is perorally administered to a human subject.

The mass ratio of pitavastatin contained in the composition (A) to pitavastatin contained in the enteric composition (B) varies in accordance with the percentage of release of pitavastatin from the composition (A) in the stomach. When the percentage of release is 85 mass% or more, the preferable ratio is within a range of 1 : 1 to 1 : 40, more preferably 1 : 1 to 1 : 20, particularly preferably 1 : 1 to 1 : 7. When the percentage of release is at least 60 mass% and less than 85 mass%, the preferable ratio is within a range of 15 : 1 to 1 : 30, more preferably 10 : 1 to 1 : 20, particularly preferably 5 : 1 to 1 : 15. When the percentage of release is at least 30 mass% and less than 60 mass%, the preferable ratio is within a range of 30 : 1 to 1 : 20, more preferably 20 : 1 to 1 : 15, particularly preferably 15 : 1 to 1 : 10.

The controlled release pharmaceutical composition of the present invention comprises the composition (A) and the enteric composition (B). Examples of more specific forms of the composition include drug preparations obtained by mixing the composition (A) and the enteric composition (B); drug preparations obtained from the composition (A) and the enteric composition (B) which are individually prepared; and drug preparations obtained by covering/coating the enteric composition (B) with the composition (A).

The controlled release pharmaceutical composition of the present invention is preferably in the drug form of peroral preparation such as tablets, granules, or capsules. Examples of tablets include uncoated tablets, chewable tablets, film-coated tablets, sugar-coated tablets, nucleated core-shell tablets, and multiple-layer tablets. Examples of granules include powders and fine granules. Examples of capsules include hard capsules and soft capsules.

No particular limitation is imposed on the amount of pitavastatin incorporated into the controlled release pharmaceutical composition of the present invention, and the preferable amount is within a range of 0.01 to 60 mass%.

The amounts of ordinary bases, sustained release components, and enteric components incorporated into the controlled release pharmaceutical composition of the present invention vary depending on the types of these additives, design of release control of drugs, etc. However, the amount of each additive preferably is within a range of 0.01 to 80 mass%, more preferably 0.1 to 50 mass%, particularly preferably 1.0 to 30 mass%.

The basis substance is preferably added in an amount for attaining the aforementioned pH value; e.g., 0.01 to 20 mass%. The diluents, binders, disintegrants, lubricants, sweetening agents, flavoring agents, and coloring agents are preferably added in amounts of 0 to 90 mass%, 0 to 20 mass%, 0 to 30 mass%, 0 to 20 mass%, 0 to 30 mass%, 0 to 30 mass%, and 0 to 5 mass%, respectively.

Examples of preferred drug forms of the controlled release pharmaceutical composition of the present invention for attaining a satisfactory blood level of pitavastatin include the following forms:
(1) a granular product obtained by coating the composition (A) on the enteric composition (B);
(2) a granular product obtained by mixing the enteric composition (B) and the composition (A) described in (1) with a pharmaceutical additive such as diluents, binders, disintegrants, etc., and forming the mixture;
(3) a granular product exhibiting at least two types of dissolution patterns and containing at least the composition (A) and the enteric composition (B);
(4) a capsule product filled with any of the granular products of the aforementioned (1), (2), and/or (3);
(5) a capsule product filled with the capsule product formed in (4) and the composition (A);
(6) a tablet product obtained by mixing any of the granular products of the aforementioned (1), (2), and/or (3) with at least one pharmaceutical additive selected from among a diluent, a binder, and a disintegrant, and forming the mixture;
(7) a multiple-layer tablet product obtained by adding at least one pharmaceutical additive selected from among a diluent, a binder, and a disintegrant to the granular product (3) exhibiting at least two types of dissolution patterns, and forming the mixture;
(8) a multiple-layer tablet product obtained by separately forming layers of the enteric composition (B) and a layer of the composition (A);
(9) a nucleated tablet product obtained by forming a core tablet of the enteric composition (B) and an outer layer of the composition (A); and
(10) a nucleated tablet product obtained by coating core tablets of a tablet product of (8) and/or (9) with the composition (A) for forming an outer layer.

The daily dose of pitavastatin contained in the thus-obtained controlled release pharmaceutical composition of the present invention is adjusted to 0.5 to 64 mg, preferably 1 to 32 mg, particularly preferably 4 to 16 mg.

### Examples

The present invention will next be described in more detail by way of examples, which should not be construed as limiting the invention thereto.

### (1) Example 1

**Table 2**

| Components | | | Formulation (mg) |
|---|---|---|---|
| Enteric granular product A | Granular product A | Purified sucrose spheres | 114.00 |
| | | Pitavastatin calcium salt | 12.00 |
| | | Sucrose | 30.75 |
| | | Hydroxypropylmethylcellulose (Registered trade mark: TC-5R) | 18.00 |
| | | Magnesium alumino metasilicate | 2.40 |
| | | Talc | 0.60 |
| | | Triethyl citrate | 2.25 |
| | | Subtotal | 180.00 |
| | Methacrylate·methyl methacrylate copolymer (Registered trade mark: Eudragit L) | | 41.58 |
| | Talc | | 8.28 |
| | Triethyl citrate | | 4.14 |
| | Subtotal | | 234.00 |
| Pitavastatin calcium salt | | | 4.00 |
| Sucrose | | | 10.25 |
| Hydroxypropylmethylcellulose (Registered trade mark: TC-5R) | | | 6.00 |
| Magnesium alumino metasilicate | | | 0.80 |
| Talc | | | 0.20 |
| Triethyl citrate | | | 0.75 |
| Total | | | 256.00 |

To purified water (3000.0 g), pitavastatin calcium salt (240.0 g), sucrose (615.0 g), hydroxypropylmethylcellulose (registered trade mark: TC-5R) (360.0 g), magnesium alumino metasilicate (48.0 g), talc (12.0 g) and triethyl citrate (45.0 g) were added, and these components were dissolved/dispersed by use of a disperser, to thereby prepare a drug-containing coating solution.

Purified sucrose spheres (2280.0 g) were fed to a fluid bed granulator with bottom rotor, and heated to about 36°C. The purified sucrose spheres were coated with the drug-containing coating solution so as to form a drug layer on each granule, to thereby produce a granular product A (3600.0 g).

To ethanol (6912.0 g), methacrylate·methyl methacrylate copolymer (registered trade mark: Eudragit L) (831.6 g), triethyl citrate (82.8 g) and talc (165.6 g) were added, and these components were dissolved/dispersed by use of a disperser, to thereby prepare an enteric coating solution.

The granular product A (3600.0 g) was fed to fluid bed granulator with bottom rotor, and heated to about 36°C. The granular product A was coated with the enteric coating solution so as to form enteric coating film on the granules, to thereby produce an enteric granular product A (4680.0 g).

To purified water (1000.0 g), pitavastatin calcium salt (80.0 g), sucrose (205.0 g), hydroxypropylmethylcellulose (registered trade mark: TC-5R) (120.0 g), magnesium alumino metasilicate (16.0 g), talc (4.0 g) and triethyl citrate (15.0 g) were added, and these components were dissolved/dispersed by use of a disperser, to thereby prepare a drug-containing coating solution.

The enteric granular product A (4680.0 g) was fed to a fluid bed granulator with bottom rotor, and heated to about 36°C. The enteric granular product A was coated with the drug-containing coating solution so as to form a drug layer on the granules, to thereby produce a granular product (5120.0 g). The product was divided into 20,000 sachets, each containing 256.0 mg.

### (2) Example 2

**Table 3**

| Components | | | Formulation (mg) |
|---|---|---|---|
| Enteric granular product | Granular product | Granular product A | 30.00 |
| | | Ethyl acrylate·methyl methacrylate·(chlorotrimethylammonio) copolymer ethyl methacrylate copolymer (Registered trade mark: Eudragit RS) | 12.40 |
| | | Hydroxypropylcellulose | 3.10 |
| | | Talc | 0.95 |
| | | Triethyl citrate | 1.55 |
| | | Subtotal Subtotal | 48.00 |
| | Pitavastatin calcium salt | | 4.00 |
| | Sucrose | | 10.25 |
| | Hydroxypropylmethylcellulose (Registered trade mark: TC-5R) | | 6.00 |
| | Magnesium alumino metasilicate | | 0.80 |
| | Talc | | 0.20 |
| | Triethyl citrate | | 0.75 |
| | Hydroxypropylmethylcellulose phthalate | | 16.17 |
| | Talc | | 3.22 |
| | Triethyl citrate | | 1.61 |
| | Subtotal | | 91.00 |
| Pitavastatin calcium salt | | | 4.00 |
| Sucrose | | | 10.25 |
| Hydroxypropylmethylcellulose (Registered trade mark: TC-5R) | | | 6.00 |
| Magnesium alumino metasilicate | | | 0.80 |
| Talc | | | 0.20 |
| Triethyl citrate | | | 0.75 |
| Total | | | 113.00 |

A granular product A (300.0 g) was produced in a manner similar to that described in relation to Example 1.

To ethanol (1200.0 g), ethyl acrylate·methyl methacrylate·(chlorotrimethylammmonio)ethyl methacrylate copolymer (registered trade mark: Eudragit RS) (124.0 g), hydroxypropylcellulose (31.0 g), triethyl citrate (15.5 g) and talc (9.5 g) were added, and these components were dissolved/dispersed by use of a disperser, to thereby prepare a coating solution.

The granular product A (300.0 g) was fed to a fluid bed granulator with bottom rotor, and heated to about 36°C. The granular product A was coated with the above coating solution, to thereby produce a granular product (480.0 g).

To purified water (500.0 g), pitavastatin calcium salt (40.0 g), sucrose (102.5 g), hydroxypropylmethylcellulose (registered trade mark: TC-5R) (60.0 g), magnesium alumino metasilicate (8.0 g), talc (2.0 g) and triethyl citrate (7.5 g) were added, and these components were dissolved/dispersed by use of a disperser, to thereby prepare a drug-containing coating solution.

To ethanol (1312.5 g), hydroxypropylmethylcellulose phthalate (161.7 g), triethyl citrate (16.1 g) and talc (32.2 g) were added, and these components were dissolved/dispersed by use of a disperser, to thereby prepare an enteric coating solution.

The granular product (480.0 g) were fed a fluid bed granulator with bottom rotor, and heated to about 36°C. The granular product was sequentially coated with the drug-containing coating solution and the enteric coating solution so as to form enteric coating films on the granules, to thereby produce an enteric granular product (910.0 g).

To purified water (500.0 g), pitavastatin calcium salt (40.0 g), sucrose (102.5 g), hydroxypropylmethylcellulose (registered trade mark: TC-5R) (60.0 g), magnesium alumino metasilicate (8.0 g), talc (2.0 g) and triethyl citrate (7.5 g) were added, and these components were dissolved/dispersed by use of a disperser, to thereby prepare a drug-containing coating solution.

The enteric granular product (910.0 g) was fed to a fluid bed granulator with bottom rotor, and heated to about 36°C. The enteric granular product was coated with the drug-containing coating solution so as to form a drug layer on the granules, to thereby produce a granular product (1130.0 g). The product was divided into 10,000 sachets, each containing 113.0 mg.

### (3) Example 3

**Table 4**

| Components | | Formulation (mg) |
|---|---|---|
| Granular product | Granular product A | 180.00 |
| | Ethylcellulose | 10.00 |
| | Hydroxypropylmethylcellulose (Registered trade mark: TC-5R) | 32.50 |
| | Talc | 8.25 |
| | Triethyl citrate | 3.25 |
| | Subtotal | 234.00 |
| Enteric granular product | Granular product A | 60.00 |
| | Methacrylate·methyl methacrylate copolymer (Registered trade mark: Eudragit S) | 13.86 |
| | Talc | 2.76 |
| | Triethyl citrate | 1.38 |
| | Subtotal | 78.00 |
| Total | | 312.00 |

A granular product A (2400.0 g) was produced in a manner similar to that described in relation to Example 1.

Hydroxypropylmethylcellulose (registered trade mark: TC-5R) (325.0 g) was dissolved in purified water (1500 g) by use of a disperser. Ethylcellulose (100.0 g), triethyl citrate (32.5 g) and talc (82.5 g) were dissolved/dispersed in ethanol (4000.0 g) by use of a disperser. The two solutions were mixed, to thereby prepare a coating solution.

A portion (1800.0 g) of the granular product A was fed to a fluid bed granulator with bottom rotor, and heated to about 36°C. The granular product A was coated with the coating solution, to thereby produce a granular product (2340.0 g).

To ethanol (1152.0 g), methacrylate·methyl methacrylate copolymer (registered trade mark: Eudragit S) (138.6 g), triethyl citrate (13.8 g) and talc (27.6 g) were added, and these components were dissolved/dispersed by use of a disperser, to thereby prepare an enteric coating solution.

A portion (600.0 g) of the granular product A was fed to a fluid bed granulator with bottom rotor, and heated to about 36°C. The granular product A was coated with the enteric coating solution so as to form enteric coating film on the granules, to thereby produce an enteric granular product (780.0 g).

The granular product (2340.0 g) and the enteric granular product (780.0 g) were mixed, and the mixture was divided into 10,000 sachets, each containing 312.0 mg.

### (4) Example 4

**Table 5**

| Components | | Formulation (mg) |
|---|---|---|
| Granular product A | | 60.00 |
| Granular product | Pitavastatin calcium salt | 4.00 |
| | Ethyl acrylate·methyl methacrylate·(chlorotrimethylammonio)ethyl methacrylate copolymer (Registered trade mark: Eudragit RS) | 65.52 |
| | Lactose | 43.68 |
| | Low-substituted hydroxypropylcellulose | 6.00 |
| | Magnesium alumino metasilicate | 0.80 |
| | Subtotal | 120.00 |
| Enteric granular product | Granular product A | 90.00 |
| | Hydroxypropylmethylcellulose acetate succinate | 20.79 |
| | Talc | 4.14 |
| | Triethyl citrate | 2.07 |
| | Subtotal | 117.00 |
| Total | | 297.00 |

A granular product A (2250.0 g) was produced in a manner similar to that described in relation to Example 1.

A pitavastatin calcium salt (60.0 g), ethyl acrylate·methyl methacrylate·(chlorotrimethylammonio) ethyl methacrylate copolymer (registered trade mark: Eudragit RS) (982.8 g), lactose (655.2 g), low-substituted hydroxypropylcellulose (90.0 g) and magnesium alumino metasilicate (12.0 g) were mixed, to thereby prepare a uniform powder mixture. A suitable amount of ethanol was added thereto, and the resultant mixture was granulated by a high-shear granulator, to thereby produce a granular product (1800.0 g).

To ethanol (5225.5 g), hydroxypropylmethylcellulose acetate succinate (311.85 g), triethyl citrate (31.05 g) and talc (62.1 g) were added, and these components were dissolved/dispersed by use of a disperser, to thereby prepare an enteric coating solution.

A portion (1350.0 g) of the granular product A was fed a fluid bed granulator with bottom rotor, and heated to about 36°C. The granular product A was coated with the enteric coating solution so as to form enteric coating film on the granules, to thereby produce an enteric granular product (1755.0 g).

The granular product A (900.0 g), the granular product (1800.0 g) and the enteric granular product (1755.0 g) were mixed, and the mixture was divided into 15,000 sachets, each containing 297.0 mg.

### (5) Example 5

**Table 6**

| Components | Formulation (mg) |
|---|---|
| Granular product A | 60.00 |
| Enteric granular product A | 234.00 |
| Gelatin capsule #1 | - |
| Total | 294.00 |

A granular product A (1200.0 g) was produced in a manner similar to that described in relation to Example 1.

An enteric granular product A (4680.0 g) was produced in a manner similar to that described in relation to Example 1.

The granular product A (1200.0 g) and the enteric granular product A (4680.0 g) were mixed, and the mixture was divided into 20,000 capsules (gelatin capsule #1), each containing 294.0 mg.

### (6) Example 6

**Table 7**

| Components | | Formulation (mg) |
|---|---|---|
| Granular product A | | 90.00 |
| Enteric granular product | Granular product A | 90.00 |
| | Methacrylate-ethyl acrylate copolymer (Registered trade mark: Eudragit L30D-55) | 20.79 |
| | Talc | 4.14 |
| | Triehtyl citrate | 2.07 |
| | Subtotal | 117.00 |
| Crystalline cellulose | | 214.62 |
| Hydroxypropylmethylcellulose (Registered trade mark: TC-5R) | | 14.70 |
| Low-substituted hydroxypropylcellulose | | 58.80 |
| Magnesium stearate | | 5.88 |
| Total | | 501.00 |

A granular product A (1800.0 g) was produced in a manner similar to that described in relation to Example 1.

To purified water (2304.0 g), methacrylate·ethyl acrylate copolymer (registered trade mark: Eudragit L30D-55) (207.9 g), triethyl citrate (20.7 g) and talc (41.4 g) were added, and these components were dissolved/dispersed by use of a disperser, to thereby prepare an enteric coating solution.

A portion (900.0 g) of the granular product A was fed to a fluid bed granulator with bottom rotor, and heated to about 36°C. The granular product A was coated with the enteric coating solution so as to form enteric coating film on the granules, to thereby produce an enteric granular product (1170.0 g).

The granular product A (900.0 g), the enteric granular product (1170.0 g), crystalline cellulose (2146.2 g), hydroxypropylmethylcellulose (registered trade mark: TC-5R) (147.0 g), low-substituted hydroxypropylcellulose (588.0 g) and magnesium stearate (58.8 g) were mixed and compressed, to thereby produce 10,000 tablets, each tablet having a weight of 501.0 mg.

### (7) Example 7

**Table 8**

| Components | | Formulation (mg) |
|---|---|---|
| Enteric granular product A | | 195.00 |
| Mixed granular product | Pitavastatin calcium salt | 4.00 |
| | Lactose | 345.00 |
| | Low-substituted hydroxypropylcellulose | 77.00 |
| | Hydroxypropylmethylcellulose (Registered trade mark: TC-5R) | 8.80 |
| | Magnesium alumino metasilicate | 0.80 |
| | Magnesium stearate | 4.40 |
| | Subtotal | 440.00 |
| Total | | 635.00 |

An enteric granular product A (1950.0 g) was produced in a manner similar to that described in relation to Example 1.

Pitavastatin calcium salt (40.0 g), lactose (3450.0 g), low-substituted hydroxypropylcellulose (770.0 g), hydroxypropylmethylcellulose(registered trade mark: TC-5R) (88.0 g) and magnesium alumino metasilicate (8.0 g) were mixed, to thereby prepare a uniform powder mixture. A suitable amount of purified water was added thereto, and the resultant mixture was granulated by a high-shear granulator. The thus-obtained granular product was mixed with magnesium stearate (44.0 g), to thereby produce a mixed granular product (4400.0 g).

The enteric granular product A (1950.0 g) and the mixed granular product (4400.0 g) were mixed and compressed, to thereby produce 10,000 tablets, each tablet having a weight of 635.0 mg.

### (8) Example 8

**Table 9**

| Components | | Formulation (mg) |
|---|---|---|
| A layer | Granular product A | 60.00 |
| | Crystalline cellulose | 43.80 |
| | Hydroxypropylmethylcellulose (Registered trade mark: TC-5R) | 3.00 |
| | Low-substituted hydroxypropylcellulose | 12.00 |
| | Magnesium stearate | 1.20 |
| | Subtotal | 120.00 |
| B layer | Enteric granular product A | 78.00 |
| | Crystalline cellulose | 56.94 |
| | Hydroxypropylmethylcellulose (Registered trade mark: TC-5R) | 3.90 |
| | Low-substituted hydroxypropylcellulose | 15.60 |
| | Magnesium stearate | 1.56 |
| | Subtotal | 156.00 |
| Total | | 276.00 |

A granular product A (600.0 g) was produced in a manner similar to that described in relation to Example 1.

An enteric granular product A (780.0 g) was produced in a manner similar to that described in relation to Example 1.

A mixture containing the granular product A (600.0 g), crystalline cellulose (438.0 g), hydroxypropylmethylcellulose (registered trade mark: TC-5R) (30.0 g), low-substituted hydroxypropylcellulose (120.0 g) and magnesium stearate(12.0 g), and another mixture containing the enteric granular product A (780.0 g), crystalline cellulose (569.4 g), hydroxypropylmethylcellulose (registered trade mark: TC-5R) (39.0 g), low-substituted hydroxypropylcellulose (156.0 g) and magnesium stearate (15.6 g) were compressed, to thereby produce 10,000 two-layer tablets, each tablet having a weight of 276.0 mg (A layer: 120.0 mg, B layer: 156.0 mg).

### (9) Example 9

**Table 10**

| Components | | Formulation (mg) |
|---|---|---|
| A layer | Mixed granular product | 440.00 |
| B layer | Pitavastatin calcium salt | 8.00 |
| | Carboxymethylethylcellulose | 101.01 |
| | Lactose | 67.36 |
| | Low-substituted hydroxypropylcellulose | 20.00 |
| | Magnesium alumino metasilicate | 1.60 |
| | Magnesium stearate | 2.00 |
| | Subtotal | 200.00 |
| Total | | 640.00 |

A mixed granular product (4400.0 g) was produced in a manner similar to that described in relation to Example 7.

Pitavastatin calcium salt (80.0 g), carboxymethylethylcellulose (1010.4 g), lactose (673.6 g), low-substituted hydroxypropylcellulose (200.0 g) and magnesium alumino metasilicate (16.0 g) were mixed, to thereby prepare a uniform powder mixture. A suitable amount of ethanol was added thereto, and the resultant mixture was granulated by a high-shear granulator. The thus-obtained granular product was mixed with magnesium stearate (20.0 g), to thereby produce an enteric mixed granular product (2000.0 g).

The mixed granular product (4400.0 g) and the enteric mixed granular product (2000.0 g) were compressed, to thereby produce 10,000 two-layer tablets, each tablet having a weight of 640.0 mg (A layer: 440.0 mg, B layer: 200.0 mg).

### (10) Example 10

**Table 11**

| Components | | Formulation (mg) |
|---|---|---|
| A layer | Pitavastatin calcium salt | 8.00 |
| | Ethyl acrylate·methyl methacrylate (chlorotrimethylammonio)ethyl methacrylate copolymer (Registered trade mark: Eudragit RS) | 56.88 |
| | Lactose | 160.32 |
| | Low-substituted hydroxypropylcellulose | 12.00 |
| | Magnesium alumino metasilicate | 1.60 |
| | Magnesium stearate | 1.20 |
| | Subtotal | 240.00 |
| B layer | Pitavastatin calcium salt | 4.00 |
| | Carboxymethylethylcellulose | 50.52 |
| | Lactose | 33.68 |
| | Low-substituted hydroxypropylcellulose | 10.00 |
| | Magnesium alumina metasilicate | 0.80 |
| | Magnesium stearate | 1.00 |
| | Subtotal | 100.00 |
| Total | | 340.00 |

Pitavastatin calcium salt (80.0 g), ethyl acrylate·methyl methacrylate·(chlorotrimethylammonio)ethyl methacrylate copolymer (registered trade mark: Eudragit RS) (568.8 g), lactose (1603.2 g), low-substituted hydroxypropylcellulose (120.0 g) and magnesium alumino metasilicate (16.0 g) were mixed, to thereby prepare a uniform powder mixture. A suitable amount of ethanol was added thereto, and the resultant mixture was granulated by a high-shear granulator. The thus-obtained granular product was mixed with magnesium stearate (12.0 g), to thereby produce a mixed granular product (2400.0 g).

Pitavastatin calcium salt (40.0 g), carboxymethylethylcellulose (505.2 g), lactose (336.8 g), low-substituted hydroxypropylcellulose (100.0 g) and magnesium alumino metasilicate (8.0 g) were mixed, to thereby prepare a uniform powder mixture. A suitable amount of ethanol was added thereto, and the resultant mixture was granulated by a high-shear granulator. The thus-obtained granular product was mixed with magnesium stearate (10.0 g), to thereby produce an enteric mixed granular product (1000.0 g).

The mixed granular product (2400.0 g) and the enteric mixed granular product (1000.0 g) were compressed, to thereby produce 10,000 two-layer tablets, each tablet having a weight of 340.0 mg (A layer: 240.0 mg, B layer: 100.0 mg).

### (11) Example 11

**Table 12**

| Components | | Formulation (mg) |
|---|---|---|
| Core tablet | Pitavastatin calcium salt | 6.00 |
| | Lactose | 32.40 |
| | Low-substituted hydroxypropylcellulose | 4.50 |
| | Hydroxypropylmethylcellulose (Registered trade mark: TC-5R) | 0.75 |
| | Magnesium alumino metasilicate | 0.90 |
| | Magnesium stearate | 0.45 |
| | Methacrylate·methyl methacrylate copolymer (Registered trade mark: Eudragit S) | 15.40 |
| | Talc | 1.54 |
| | Triethyl citrate | 3.06 |
| | Subtotal | 65.00 |
| Outer layer | Mixed granular product | 220.00 |
| Total | | 285.00 |

Pitavastatin calcium salt (60.0 g), lactose (324.0 g), low-substituted hydroxypropylcellulose (45.0 g), hydroxypropylmethylcellulose (registered trade mark: TC-5R) (7.5 g) and magnesium alumino metasilicate (9.0 g) were mixed, to thereby prepare a uniform powder mixture. A suitable amount of purified water was added thereto, and the resultant mixture was granulated by a high-shear granulator. The thus-obtained granular product and magnesium stearate (4.5 g) were mixed and compressed, to thereby produce 10,000 uncoated tablets (total 450.0 g), each tablet having a weight of 45.0 mg.

To ethanol (2560.0 g), methacrylate·methyl methacrylate copolymer (registered trade mark: Eudragit S) (154.0 g), triethyl citrate (30.6 g) and talc (15.4 g) were added, and these components were dissolved/dispersed by use of a disperser, to thereby prepare an enteric coating solution.

The uncoated tablets (450.0 g) were fed to a coating pan, and heated to 40 to 45°C. The uncoated tablets were coated with the enteric coating solution so as to form enteric coating film on the tablets, to thereby produce enteric tablets (650.0 g, 10,000 tablets).

A mixed granular product (2200.0 g) was produced in a manner similar to that described in relation to Example 7.

The enteric tablets (650.0 g) for forming core tablets and the mixed granular product (2200.0 g) for forming an outer layer were compressed, to thereby produce 10,000 core-shell tablets, each tablet having a weight of 285.0 mg.

### (12) Example 12

**Table 13**

| Components | | Formulation (mg) |
|---|---|---|
| Core tablet | Pitavastatin calcium salt | 9.00 |
| | Methacrylate·methyl methacrylate copolymer (Registered trade mark: Eudragit S) | 38.07 |
| | Lactose | 25.38 |
| | Low-substituted hydroxypropylcellulose | 4.85 |
| | Magnesium alumino metasilicate | 1.80 |
| | Magnesium stearate | 0.90 |
| | Subtotal | 80.00 |
| Outer layer | Mixed granular product | 330.00 |
| Total | | 410.00 |

A mixed granular product (3300.0 g) was produced in a manner similar to that described in relation to Example 7.

Pitavastatin calcium salt (90.0 g), methacrylate·methyl methacrylate copolymer (registered trade mark: Eudragit S) (380.7 g), lactose (253.8 g), low-substituted hydroxypropylcellulose (48.5 g) and magnesium alumino metasilicate (18.0 g) were mixed, to thereby prepare a uniform powder mixture. A suitable amount of ethanol was added thereto, and the resultant mixture was granulated by a high-shear granulator. The thus-obtained granular product and magnesium stearate (9.0 g) were mixed and compressed, to thereby produce 10,000 enteric tablets (800.0 g), each tablet having a weight of 80.0 mg.

The enteric tablets (800.0 g) for forming core tablets and the mixed granular product (3300.0 g) for forming an outer layer were compressed, to thereby produce 10,000 core-shell tablets, each tablet having a weight of 410.0 mg.

### (13) Example 13

**Table 14**

| Components | | | Formulation (mg) |
|---|---|---|---|
| Core tablet | Enteric granular product | Purified sucrose spheres | 40.00 |
| | | Pitavastatin calcium salt | 12.00 |
| | | Sucrose | 14.35 |
| | | Hydroxypropylmethylcellulose (Registered trade mark: TC-5R) | 8.40 |
| | | Magnesium alumino metasilicate | 2.40 |
| | | Talc | 0.60 |
| | | Triethyl citrate | 2.25 |
| | | Cellulose acetate phthalate | 18.48 |
| | | Talc | 3.68 |
| | | Triethyl citrate | 1.84 |
| | | Subtotal | 104.00 |
| | Crystalline cellulose | | 75.92 |
| | Hydroxypropylmethylcellulose (Registered trade mark: TC-5R) | | 5.20 |
| | Low-substituted hydroxypropylcellulose | | 20.80 |
| | Magnesium stearate | | 2.08 |
| | Subtotal | | 208.00 |
| Outer layer | Mixed granular product | | 440.00 |
| Total | | | 648.00 |

To purified water (4000.0 g), pitavastatin calcium salt (120.0 g), sucrose (143.5 g), hydroxypropylmethylcellulose (registered trade mark: TC-5R) (84.0 g), magnesium alumino metasilicate (24.0 g), talc (6.0 g) and triethyl citrate (22.5 g) were added, and these components were dissolved/dispersed by use of a disperser, to thereby prepare a drug-containing coating solution.

Purified sucrose spheres (400.0 g) were fed to a fluid bed granulator with bottom rotor, and heated to about 36°C. The purified sucrose spheres were coated with the drug-containing coating solution so as to form a drug layer on each granule, to thereby produce a granular product (800.0 g).

To acetone (2304.0 g), cellulose acetate phthalate (184.8 g), triethyl citrate (18.4 g), and talc (36.8 g) were added, and these components were dissolved/dispersed by use of a disperser, to thereby prepare an enteric coating solution.

The granular product (800.0 g) was fed to a fluid bed granulator with bottom rotor, and heated to about 36°C. The granular product was coated with the enteric coating solution so as to form enteric coating film on the granules, to thereby produce an enteric granular product (1040.0 g).

The enteric granular product (1040.0 g), crystalline cellulose (759.2 g), hydroxypropylmethylcellulose (registered trade mark: TC-5R) (52.0 g), low-substituted hydroxypropylcellulose (208.0 g), and magnesium stearate (20.8 g) were mixed and compressed, to thereby produce 10,000 core tablets (2080.0 g), each tablet having a weight of 208.0 mg.

A mixed granular product (4400.0 g) was produced in a manner similar to that described in relation to Example 7.

The core tablet (2080.0 g) and the mixed.granular product (4400.0 g) for forming an outer layer were compressed, to thereby produce 10,000 core-shell tablets, each tablet having a weight of 648.0 mg.

### Production Examples 1 and 2

Pitavastatin-controlled-release tablets can be produced in accordance with the formulations shown in Table 15. Specifically, after preparation of core tablets, a drug-controlled-release coating layer is formed on the core tablets. Methacrylate·methyl methacrylate copolymer (trade name: Eudragit L, available from: Higuchi Inc.) is an enteric polymer which dissolves at a pH of 6 to 14, and ethylcellulose (trade name: Ethocel, product of Dow Chemical) is a sustained-release polymer which is insoluble regardless of pH. The tablets obtained in Production Examples are pitavastatin-controlled-release tablets.

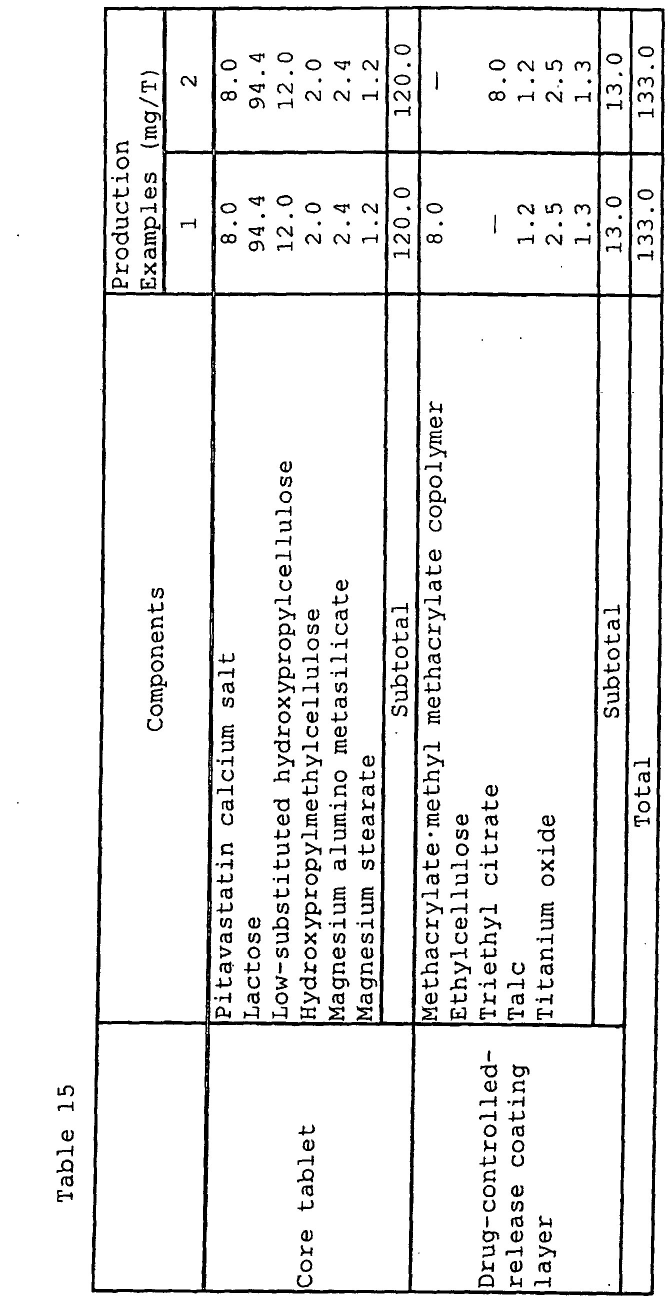

### Example 14

The tablets obtained in Production Example 1 are further coated with a pitavastatin-containing composition of Table 16. The tablet product of Example 14, which is formed of an outermost layer (immediately release portion) and an inner layer (controlled release portion) in combination, maintains reliable release of pitavastatin immediately after administration over a long period of time.

**Table 16**

| | Components | Example 14 (mg/T) |
|---|---|---|
| Inner layer | Tablets (Production Example 1) | 133.0 |
| Outermost layer | Pitavastatin calcium salt | 8.0 |
| | Lactose | 304.0 |
| | Low-substituted hydroxypropylcellulose | 36.0 |
| | Hydroxypropylmethylcellulose | 6.0 |
| | Magnesium alumino metasilicate | 2.4 |
| | Magnesium stearate | 3.6 |
| | Subtotal | 360.0 |
| Total | | 493.0 |

### Example 15

A pitavastatin-controlled-release granular product can be produced in accordance with the formulation shown in Table 17. Specifically, after preparation of core granules, a drug-controlled-release coating layer is formed on the core granules. The core granules and the drug-controlled-release-layer-coated core granules are mixed, to thereby yield a controlled release granular product.

**Table 17**

| | Components | Example 15 (mg/day) |
|---|---|---|
| Core granular product | Pitavastatin calcium salt | 8.0 |
| | D-mannitol | 334.0 |
| | Calcium Carboxymethylcellulose | 21.0 |
| | Hydroxypropylcellulose | 21.0 |
| | Magnesium alumino metasilicate | 16.0 |
| | Subtotal | 400.0 |
| Drug-controlled-release coating layer | Methacrylate·methyl methacrylate copolymer | 54.0 |
| | Triethyl citrate | 12.0 |
| | Talc | 50.0 |
| | Titanium oxide | 4.0 |
| | Subtotal | 120.0 |

### Example 16

The procedure of Example 15 is repeated, except that the formulation of Table 18 is employed, to thereby yield a granular product whose release is slower than that of the product of Example 15.

**Table 18**

| | Components | Example 16 (mg/day) |
|---|---|---|
| Core granular product | Pitavastatin calcium salt | 8.0 |
| | D-mannitol | 334.0 |
| | Calcium Carboxymethylcellulose | 21.0 |
| | Hydroxypropylcellulose | 21.0 |
| | Magnesium alumino metasilicate | 16.0 |
| | Subtotal | 400.0 |
| Drug-controlled-release coating layer | Methacrylate·methyl methacrylate | 54.0 |
| | copolymer | |
| | Ethylcellulose | 27.0 |
| | Triethyl citrate | 18.0 |
| | Talc | 75.0 |
| | Titanium oxide | 6.0 |
| | Subtotal | 180.0 |

A pitavastatin-controlled-release capsule product can be obtained by mixing core granules of Table 17; drug-controlled-release layer-coated granules of Table 17; and drug-controlled-release layer-coated granules of Table 18 and by charging the mixture in capsules.

A pitavastatin-controlled-release tablet product can be obtained by mixing core granules of Table 17; drug-controlled-release layer-coated granules of Table 17; and drug-controlled-release layer-coated granules of Table 18 and by compressed the mixture.

A pitavastatin-controlled-release multiple-layer tablet product can be obtained by individually compressing a layer of core granules of Table 17 and a layer of drug-controlled-release layer-coated granules of Table 17.

### Production Example 3

A pitavastatin-controlled-release tablet product can be produced in accordance with the formulation shown in Table 19. Specifically, after preparation of core tablets of Table 19, an outer layer component shown in Table 19 is formed on the core tablets by compressing, to thereby yield core-shell tablets.

**Table 19**

| Components | | Production Example 3 (mg/T) |
|---|---|---|
| Core tablet | Pitavastatin calcium salt | 8.0 |
| | Lactose | 46.4 |
| | Crospovidone | 20.0 |
| | Hydroxypropylmethylcellulose | 2.0 |
| | Magnesium alumino metasilicate | 2.4 |
| | Ethyl acrylate·methyl methacrylate | 20.0 |
| | (chlorotrimethylammonio) ethyl methacrylate copolymer^{*1} Monoglyceride | 20.0 |
| | Magnesium stearate | 1.2 |
| Total | | 120.0 |
| Outer layer | Pitavastatin calcium salt | 8.0 |
| | Lactose | 304.0 |
| | Low-substituted hydroxypropylcellulose | 36.0 |
| | Hydroxypropylmethylcellulose | 6.0 |
| | Magnesium alumino metasilicate | 2.4 |
| | Magnesium stearate | 3.6 |
| | Subtotal | 360.0 |

| | | |
|---|---|---|
| *1: Eudragit RS sold by Higuchi Inc. | | |

### Comparative Example 1

**Table 20**

| Components | Formulation (mg) |
|---|---|
| Pitavastatin calcium salt | 16.00 |
| Lactose | 86.40 |
| Low-substituted hydroxypropylcellulose | 12.00 |
| Hydroxypropylmethylcellulose (Registered trade mark: TC-5R) | 2.00 |
| Magnesium alumino metasilicate | 2.40 |
| Magnesium stearate | 1.20 |
| Total | 120.00 |

The method described in Example 5 of WO 97/23200 was employed.

Pitavastatin calcium salt (400.0 g), lactose (2160.0 g), low-substituted hydroxypropylcellulose (300.0 g), hydroxypropylmethylcellulose (registered trade mark: TC-5R) (50.0 g) and magnesium alumino metasilicate (60.0 g) were mixed, to thereby prepare a uniform powder mixture. Purified water (594.0 g) was added thereto, and the mixture was granulated by a high-shear granulator. The thus-obtained granular product and magnesium stearate (30.0 g) were mixed and compressed, to thereby produce 25,000 tablets, each tablet having a weight of 120.0 mg.

### Comparative Example 2

**Table 21**

| Components | Formulation (mg) |
|---|---|
| Pitavastatin calcium salt | 16.00 |
| Lactose | 48.00 |
| Hydroxypropylmethylcellulose (Registered trade mark: Methocel K100LV) | 80.00 |
| Magnesium alumino metasilicate | 6.40 |
| Low-substituted hydroxypropylcellulose | 8.00 |
| Magnesium stearate | 1.60 |
| Total | 160.00 |

Pitavastatin calcium salt (320.0 g), lactose (960.0 g), hydroxypropylmethylcellulose (registered trade mark: Methocel K100LV) (1600.0 g), magnesium alumino metasilicate (128.0 g) and low-substituted hydroxypropylcellulose (160.0 g) were mixed, to thereby prepare a uniform powder mixture. Purified water (1108.8 g) was added thereto, and the resultant mixture was granulated by a high-shear granulator.
The thus-obtained granular product and magnesium stearate (32.0 g) were mixed and compressed, to thereby produce 20,000 tablets, each tablet having a weight of 160.0 mg.

### Test Example 1 Dissolution test

The dissolution property of pitavastatin from drug preparations; i.e., a drug preparation of Example 5 (composition (A) and enteric composition (B)); that of Comparative Example 1 (ordinary preparation); and that of Comparative Example 2 (sustained release preparation) was investigated in accordance with the following method.

### Dissolution test (Japanese Pharmacopoeia General Test Methods, Dissolution Test Method No. 2 (Paddle method)).

2.0 g of sodium chloride were dissolved in 7.0 mL of hydrochloric acid (36% HCl content) and some water, and water was added, to thereby prepare 1,000 mL of artificial gastric juice medium (pH: 1.2).

Water and 118 mL of 0.2 mol/L sodium hydroxide test solution were added to 250 mL of 0.2 mol/L potassium dihydrogen phosphate test solution, to thereby prepare 1,000 mL of artificial intestinal juice medium (pH: 6.8).

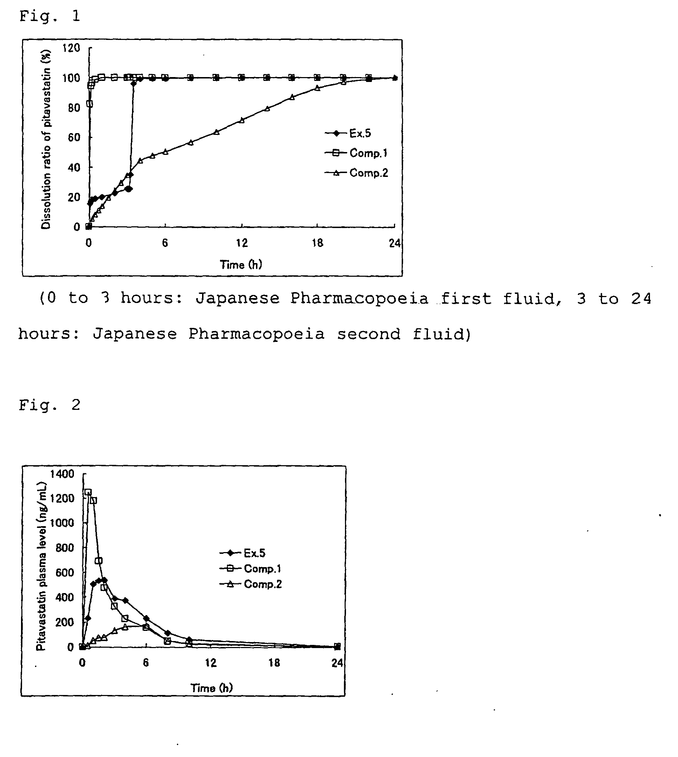

Each of drug preparations of Example 5 and Comparative Examples 1 and 2 was added to the artificial gastric juice medium (900 mL) (pH: 1.2), and the medium was stirred at 37 ± 1°C with a paddle rotation of 100 rpm for three hours after starting of the test. Immediately thereafter, the dissolution test medium was changed to the artificial intestinal juice medium (900 mL) (pH: 6.8), and the dissolution test was continued for 21 hours at 37 ± 1°C with a paddle rotation of 100 rpm. The amount of dissolved drug was determined. A sample solution sampled at each timing was filtered by means of a membrane filter made of cellulose acetate (pore size: 0.45 µm, DISMIC-25cs, product of Toyo Roshi), and the percent of dissolution of pitavastatin was determined with high performance liquid chromatography by use of a reverse-phase column (Develosil ODS-HG-5, product of Nomura Chemical). The results are shown in Fig. 1.

### Test Example 2 Absorption test

Each of the drug preparations prepared in Example 5, Comparative Example 1, and Comparative Example 2 was orally administered to an HRA beagle dog (body weight: about 10 kg) under fasting. After administration, blood was collected at different points in time for 24 hours, and the collected blood was subjected to centrifugation, whereby blood pitavastatin level was determined with high performance liquid chromatography. The results are shown in Table 22 and Fig. 2. The pharmacokinetic values are shown in Table 23. The results show that the controlled release drug preparation of the present invention reduces Cₘₐₓ by 50% as compared with the ordinary drug preparation, while providing equivalent AUC comparable to that of the ordinary drug preparation. By contrast, in the case of the sustained release drug with zero-order release, Cₘₐₓ and AUC are both significantly reduced as compared with the ordinary drug preparation. Therefore, the controlled release drug preparation of the present invention was found to be safe and highly effective.

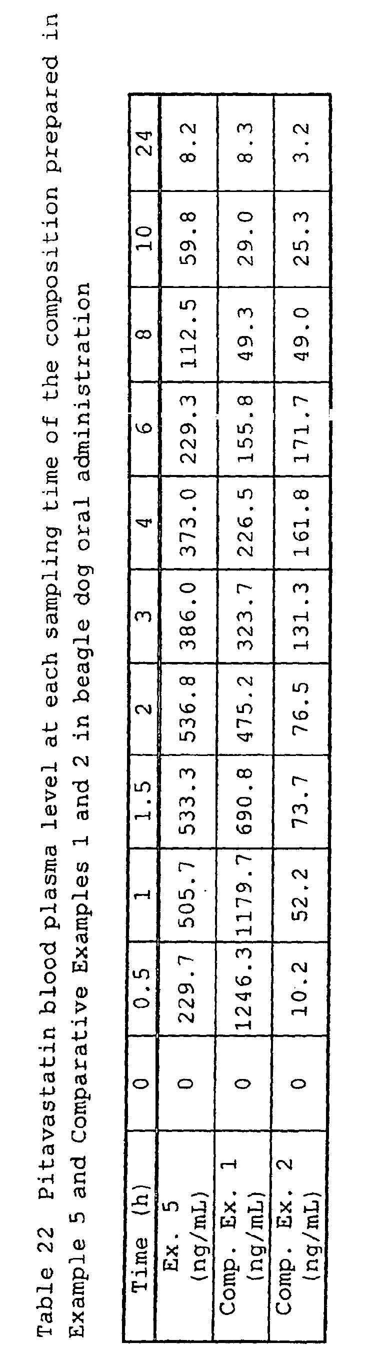

**Table 23**

| Poitavastatin pharmacokinetic value of the compositions prepared in Example 5 and Comparative Examples 1 and 2 in beagle dog oral administration | | | |
|---|---|---|---|
| | Tₘₐₓ (h) | Cₘₐₓ (ng/mL) | AUC (ng·hr/mL) |
| Example 5 | 1.8 | 748.0 | 3202 |
| Comp. Ex. 1 | 0.7 | 1434.8 | 3279 |
| Comp. Ex. 2 | 4.7 | 210.5 | 1166 |

### Industrial Applicability

By use of the controlled release pharmaceutical composition of the present invention, the blood level of pitavastatin can be maintained at an appropriate level immediately after administration over a long period of time. Thus, highly safety and effective treatment of hypercholesterolemia can be performed.

## Claims

1. A controlled release pharmaceutical composition **characterized by** comprising a composition (A) which contains pitavastatin, a salt thereof, or an ester thereof and which initiates release thereof at least in the stomach, and an enteric composition (B) which contains pitavastatin, a salt thereof, or an ester thereof.

2. A controlled release pharmaceutical composition as described in claim 1, wherein the composition (A) releases in the stomach at least 30 mass% pitavastatin, a salt thereof, or an ester thereof contained in the composition (A).

3. A controlled release pharmaceutical composition as described in claim 1, which has a ratio by mass of pitavastatin, a salt thereof, or an ester thereof contained in the composition (A) to pitavastatin, a salt thereof, or an ester thereof contained in the enteric composition (B) falling within a range of 30 : 1 to 1 : 40.

4. A controlled release pharmaceutical composition as described in claim 1, wherein the composition (A) releases in the stomach at least 85 mass% pitavastatin, a salt thereof, or an ester thereof contained in the composition (A), and the controlled release pharmaceutical composition has a ratio by mass of pitavastatin, a salt thereof, or an ester thereof contained in the composition (A) to pitavastatin, a salt thereof, or an ester thereof contained in the enteric composition (B) falling within a range of 1 : 1 to 1 : 40.

5. A controlled release pharmaceutical composition as described in claim 1, wherein the composition (A) releases in the stomach at least 60 mass% and less than 85 mass% pitavastatin, a salt thereof, or an ester thereof contained in the composition (A), and the controlled release pharmaceutical composition has a ratio by mass of pitavastatin, a salt thereof, or an ester thereof contained in the composition (A) to pitavastatin, a salt thereof, or an ester thereof contained in the enteric composition (B) falling within a range of 15 : 1 to 1 : 30.

6. A controlled release pharmaceutical composition as described in claim 1, wherein the composition (A) releases in the stomach at least 30 mass% and less than 60 mass% pitavastatin, a salt thereof, or an ester thereof contained in the composition (B), and the controlled release pharmaceutical composition has a ratio by mass of pitavastatin, a salt thereof, or an ester thereof contained in the composition (A) to pitavastatin, a salt thereof, or an ester thereof contained in the enteric composition (B) falling within a range of 30 : 1 to 1 : 20.

7. A controlled release pharmaceutical composition as described in any one of claims 1 to 6, wherein the enteric composition (B) is obtained by coating pitavastatin, a salt thereof, or an ester thereof or a composition containing the same with a component which dissolves at a pH of 3 or higher or by mixing pitavastatin, a salt thereof, or an ester thereof with a component which dissolves at a pH of 3 or higher.
